# EUROPEAN PATENT APPLICATION

(11) **EP 4 230 207 A1**
(43) Date of publication of application: **23.08.2023**
(21) Application number: 22305174.9
(22) Date of filing: 16.02.2022
(51) Int. Cl.: A61K 31/4965, A61K 9/00, A61K 31/55, A61P 25/00, A61P 25/02

(54) **AMILORIDE FOR USE IN THE TOPICAL TREATMENT OF SOMATOSENSORY IMPAIRMENTS**

(71) Applicant: ASSISTANCE PUBLIQUE HOPITAUX DE PARIS, 75012 Paris 12 (FR)
(72) Inventor: GRECO, Céline, 75012 PARIS (FR)
(74) Representative: Gevers & Orès

(57) **Abstract**

The present invention relates to a compound selected from amiloride, an amiloride analog and a pharmaceutically acceptable salt thereof, or a composition comprising such a compound, for use in the topical treatment and prevention of a somatosensory impairment.

## Description

The present invention relates to a compound selected from amiloride, an amiloride analog and a pharmaceutically acceptable salt thereof, or a composition comprising such a compound, for use in the topical treatment and prevention of a somatosensory impairment.

In mammals, sensations coming from the skin are mediated by the somatosensory nervous system. Primary somatosensory neurons are able to transduce a variety of stimuli into action potentials that propagate towards the central nervous system. Most of these sensory neurons are also responsive to some forms of mechanical stimuli and thus operate as mechanoreceptors. The stimulation of these mechanoreceptors generates a variety of sensations, such as touch, pressure, heat, vibration, proprioception or pain. The receptors involved in providing these tactile sensations to the central nervous system are Merkel's cells, Pacinian corpuscles, Meissner's corpuscles and Ruffini endings. Neural signals of the somatic mechanosensation involve the excitation of the mechanoreceptive sensory endings. It is thought that mechanical forces applied to the sensory endings directly open mechanosensitive ion channels that depolarize the terminal.

However, when both tactile and proprioceptive information is not conducted to the central nervous system as it is supposed to be, e.g. in chemotherapy induced neuropathy or a diabetic neuropathy, patients can experience somatosensory impairments, in particular hypoesthesia and mechanical allodynia.

Hypoesthesia is an abnormal sensory response in which sensation is reduced in one or more body parts in response to a stimulus such as touch, vibration or cold temperature. Hypoesthesia is often experienced as partial numbness or a loss or reduction of sensation in the body. Sometimes it is accompanied by a pins-and-needles tingling. It may be in one part of the body, such as the hand or the feet, along one side of the body or in multiple areas.

Patients suffering from hypoesthesia are frequently victims of proprioception disorders, such as falls, impacts against high obstacles (eg: sidewalk), burns to the hands (because they do not realize that they are putting their hand on a hot plate for example) or cuts (while cooking without noticing it). They can have difficulty holding objects without dropping them. These disabilities can last over time or even become irreversible.

Mechanical allodynia is a painful sensation caused by innocuous stimuli like light touch. Patients suffering from mechanical allodynia can have the feeling of having a permanently rolled up sock under their feet, walking on sand or walking on cotton wool.

Treatment of somatosensory impairments is aimed at targeting the more broad disease or illness that has caused the side effect of sensation loss or trouble. However, there is no efficient treatment to recover a normal perception of somatic sensation.

There thus remains a genuine need for an effective treatment of somatosensory impairments, such as hypoesthesia and mechanical allodynia.

The present invention is believed to meet such need by providing a topical composition for treating or preventing somatosensory impairments.

Amiloride is a chemical compound of formula (I):

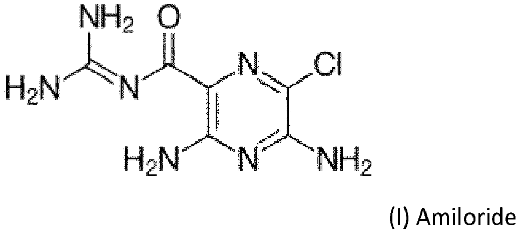

Amiloride is used clinically as a diuretic for several decades. It directly blocks the degenerin/epithelial sodium channels (DEG/ENaC) and thereby inhibits sodium reabsorption in the late distal convoluted tubules, connecting tubules and collecting ducts in the nephron. Amiloride also has an action on the heart, blocking Na+/H+ exchangers sodium-hydrogen antiporter 1 or NHE-1 and it is prescribed orally in certain forms of hypertension as well as in certain forms of heart failure or cirrhosis of the liver. Amiloride is also considered to be a reversible, pan-acid-sensing ion channel (ASIC) inhibitor. ASICs are members of the ENaC family of protein channels, and are found in the nervous system, the cardiovascular system, the gastrointestinal system and the skin.

The Inventors have in a surprising manner shown that patients treated with a topical composition of amiloride were able to recover sensations in insensitive body areas and a correct proprioception and noted the disappearance of mechanical allodynia.

Thus, the present invention concerns a compound selected from amiloride, an amiloride analog and a pharmaceutically acceptable salt thereof, for use in the topical treatment or prevention of somatosensory impairments.

The present invention further relates to a topical composition comprising a compound according to the invention as active ingredient and at least one pharmaceutically acceptable excipient, for use in the treatment or prevention of somatosensory impairments.

The present invention further relates to a method for treating and/or preventing somatosensory impairments by means of topical administration, to a patient in need thereof, of an effective amount of a compound or composition according to the invention.

The present invention further relates to the use of a compound or composition comprising a compound according to the invention for the manufacture of a topical medication for treating and/or preventing somatosensory impairments.

Without being bound by theory, the Inventors hypothesized that amiloride, due to its action on voltage-dependent calcium channels, PIEZO type mechanical currents and potassium channels, could re-excite some fibers that became hypoexcitable. Alternatively, the Inventors hypothesized that the inhibition of ASIC channels could lead to an overcompensation at nerve endings with stimulation of transient receptor potential (TRP) channels, two-pore-domain potassium (K2P) channels and/or PIEZO channels with an increased skin sensitivity.

"Amiloride" (sold under the trade name "Midamor" among others) as used herein refers to the molecule 3,5-diamino-6-chloro-N-(diaminomethylidene) pyrazine-2-carboxamide, enantiomers, racemic mixtures, polymorphs, salts, solvates, esters or hydrates thereof.

"Amiloride analog", as used herein, refer to a chemical compound having biological activities similar to those of amiloride but with a slightly altered chemical structure. The amiloride analog may be selected from, but is not limited to, the group consisting of benzamil, phenamil, 5-(N-ethyl-N-isopropyl) amiloride (EIPA), 5-(N-methyl-N-isobutyl) amiloride (MIA), 5-(N,N-hexamethylene) amiloride (HMA) and 5-(N,N-dimethyl) amiloride (DMA). The amiloride analog may be selected from the group consisting of methylated analogs of benzamil, amiloride analogs containing a ring formed on a guanidine group, amiloride analogs containing an acylguanidino group, and amiloride analogs containing a water solubilizing group formed on a guanidine group, wherein the water solubilizing group is a N,N-dimethyl amino group or a sugar group.

In the present invention, "pharmaceutically acceptable" is intended to mean that which is useful in the preparation of a pharmaceutical composition, generally safe, nontoxic and neither biologically nor otherwise undesirable, and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

Salts of amiloride include salts of acidic or basic groups present in compounds of the application. Pharmaceutically acceptable salts of amiloride include, but are not limited to, amiloride hydrochloride.

In the present invention, the term "somatosensory impairment" refers to a disorder affecting one's capability to efficiently and accurately process sensory information received by sensory receptors in the skin. Somatosensation refers to sensations perceived by the skin. Effective somatosensation means being able to distinguish temperature, feel light touch, feel pain, and being able to determine the sharpness of an object. Somatosensory impairments can affect any part of the body, for example the feet or arms.

In an embodiment, the somatosensory impairment is chosen from the group comprising hypoesthesia and mechanical allodynia.

In the present invention, the term "hypoesthesia" designates a partial or total loss of sensation in at least one area of the body. In particular, it can affect sensation of pain, temperature, touch vibration or proprioception.

In an embodiment, the invention relates to a compound or a topical composition as defined above for treating or preventing hypoesthesia.

In an embodiment, the invention relates to a compound or a topical composition as defined above for treating or preventing a proprioception disorder caused by hypoesthesia.

In the present invention, the expression "mechanical allodynia" refers to a painful sensation caused by innocuous mechanical stimuli like light touch.

In an embodiment, the invention relates to a compound or a topical composition as defined above for treating or preventing mechanical allodynia.

Causes of somatosensory impairments include, but are not limited to, a neuropathy, traumatic injuries, infections or a needle injection around a nerve.

In an embodiment, the somatosensory impairment is caused by a neuropathy.

"Neuropathy" (also referred to as "peripheral neuropathy") is a condition that involves damage to the nerves located outside of the brain and spinal cord (peripheral nerves). It often causes loss of balance and co-ordination, muscle weakness, numbness and pain, usually in the hands and feet.

In an embodiment, the somatosensory impairment is caused by a neuropathy selected from the group comprising a diabetic neuropathy, a chemotherapy-induced peripheral neuropathy or drug induced peripheral neuropathy.

"Diabetic neuropathy" is a serious diabetes complication that may affect as many as 50% of people with diabetes. Diabetic neuropathy most often damages nerves in legs and feet.

"Chemotherapy-induced peripheral neuropathy" (or "CIPN") is one of the most frequent side effects caused by antineoplastic agents. Due to its high prevalence among cancer patients, CIPN constitutes a major problem for both cancer patients and survivors. There are six main substance groups that cause damage to peripheral sensory, motor and autonomic neurons, which result in the development of CIPN: platinum-based antineoplastic agents, vinca alkaloids, epothilones (ixabepilone), taxanes, proteasome inhibitors (bortezomib) and immunomodulatory drugs (thalidomide). Clinically, sensory symptoms usually develop first, involve the feet and hands and commonly present as a typical "glove and stocking" neuropathy with the most distal parts of the limbs exhibiting the greatest deficits. The symptoms comprise numbness, tingling, altered touch sensation, impaired vibration, paresthesias and dysesthesias induced by touch and warm or cool temperatures. Moreover, painful sensations, including spontaneous burning, shooting or electric shock-like pain as well as mechanical or thermal allodynia or hyperalgesia frequently occur. In severe cases, these symptoms can progress to a loss of sensory perception.

"Drug induced peripheral neuropathy" is caused by drugs used in current clinical and that may cause a purely sensory or mixed sensorimotor neuropathy. These include antimicrobials, such as isoniazid, ethambutol, ethionamide, nitrofurantoin, and metronidazole; antineoplastic agents, particularly vinca alkaloids; cardiovascular drugs, such as perhexiline and hydrallazine; hypnotics and psychotropics, notable methaqualone; antirheumatics, such as gold, indomethacin, and chloroquine; anticonvulsants, particularly phenytoin; and other drugs.

In an embodiment, the invention relates to a compound or a topical composition as defined above for treating or preventing hypoesthesia caused by a neuropathy, preferably selected from the group comprising diabetic neuropathy, chemotherapy-induced peripheral neuropathy and drug-induced peripheral neuropathy.

In an embodiment, the invention relates to a compound or a topical composition as defined above for treating or preventing mechanical allodynia caused by a neuropathy, preferably selected from the group comprising diabetic neuropathy, chemotherapy-induced peripheral neuropathy and drug-induced peripheral neuropathy.

By "treatment" is meant, according to the present invention, the inhibition of the development of, more particularly the regression of the somatosensory impairment, preferably the disappearance of the loss or trouble of sensation or the recovery of normal or complete sensation in the body.

By "prevention" is meant, according to the present invention, to prevent or delay the appearance of the somatosensory impairment.

The treatment or prevention according to the invention applies to humans or animals.

By "topical" is meant, according to the present invention, that the compound or composition according to the invention will be administered by application on the skin surface or the mucous membranes.

The topical composition according to the invention may in particular be in any form allowing topical application, such as a cream, a gel, an ointment, a solution, a lotion, a spray, an aerosol spray, an aerosol foam or a patch. Preferably, the composition according to the invention will be in cream or gel form.

In an embodiment, the topical composition of the invention can be applied to the skin by an applicator, such as a roll-on, a stick, an impregnated wipe or an impregnated glove.

In an embodiment, the composition of the invention can also be dispensed from a pump pack or from an aerosol container.

In an embodiment, the topical composition of the invention comprises a compound according to the invention in a concentration from 0.1 to 30%, in particular from 1% to 20%, more particularly from 10% to 20%, by weight relative to the weight of the composition.

In an embodiment, the topical composition of the invention comprises a compound according to the invention in a concentration of at least 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19% , 20%, 21%, 22%, 23%, 24%, 25%, 26%, 27%, 28%, 29% or 30% by weight relative to the weight of the composition.

In an embodiment, the topical composition of the invention comprises a compound according to the invention in a concentration of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19% , 20%, 21%, 22%, 23%, 24%, 25%, 26%, 27%, 28%, 29% or 30% by weight relative to the weight of the composition.

In an embodiment, the topical composition of the invention comprises a compound according to the invention in a concentration of 5%.

In an embodiment, the topical composition of the invention comprises a compound according to the invention in a concentration of 10%.

In an embodiment, the topical composition of the invention comprises a compound according to the invention in a concentration of 15%.

In an embodiment, the topical composition of the invention is formulated into unit dose form.

The compound or topical composition according to the invention will be administered one or more times per day, the duration being variable according to the loss or reduction of sensation and easily adjustable by the person skilled in the art or the practitioner.

In an embodiment, the topical composition of the invention is applied to the affected area once daily, twice daily, three times daily, once every second day, three times weekly, twice weekly or once weekly.

In an embodiment, the topical composition comprises amiloride in a concentration of 5% for treating or preventing a somatosensory impairment caused by a neuropathy, preferably selected from the group comprising diabetic neuropathy, chemotherapy-induced peripheral neuropathy and drug-induced peripheral neuropathy.

In an embodiment, the topical composition comprises amiloride in a concentration of 10% for treating or preventing a somatosensory impairment caused by a neuropathy, preferably selected from the group comprising diabetic neuropathy, chemotherapy-induced peripheral neuropathy and drug-induced peripheral neuropathy.

In an embodiment, the topical composition comprises amiloride in a concentration of 15% for treating or preventing a somatosensory impairment caused by a neuropathy, preferably selected from the group comprising diabetic neuropathy, chemotherapy-induced peripheral neuropathy and drug-induced peripheral neuropathy.

By "pharmaceutically acceptable excipient" is meant, according to the invention, an excipient that is compatible with the other ingredients of the composition and that produces no adverse effect, allergic reaction or other undesirable reaction when it is administered to a human or an animal.

According to the invention, by "excipient" is meant in particular one or more surfactants, for example macrogols, hydroxy stearates, ethoxylated fatty acid esters, ethoxylated fatty alcohols; one or more solvents, such as for example octyldodecanol, propylene glycol dicaprylocaprate; one or more hydrosoluble polymers, for example PVP, hyaluronic acid or sodium hyaluronate; one or more thickeners such as for example natural or semisynthetic gums; one or more gelling agents, for example carbomers; one or more inorganic fillers, for example zinc oxide, talc, clays; one or more emulsifiers such as cetearyl alcohol; one or more preservatives, for example phenoxyethanol; one or more antibacterials; one or more antiseptics; one or more antioxidants, for example tocopherol acetate; one or more chelating agents, for example EDTA; one or more pigments; one or more fragrances; one or more colorants; one or more pH adjustors such as salts, acids, bases; or a mixture thereof.

In an embodiment, the topical composition of the invention further comprises a penetration enhancer.

By "penetration enhancer" is meant, according to the invention, a chemical substance that promotes the transdermal penetration of the drug applied at the surface of the skin. For example, a penetration enhancer can be selected among alcohols, amides, esters, glycols, fatty acids, pyrrolidones, sulfoxides, surfactants, terpenes, urea, cyclodextrins, water, vitamin E or phospholipids.

In an embodiment, the topical composition further comprises a base allowing transdermal diffusion of said compound. In particular, such a base may be selected from the group comprising Excipial Hydrocrème^{®}, Codexial Obase^{®}, Pentravan^{®}, Pentravan^{®} Plus, Phytobase^{®}, Lipovan^{®} and Pluronic Lecithin Organogel (PLO), preferably Pentravan^{®}, preferably Excipial Hydrocreme^{®} or Codexial Obase^{®}.

In an embodiment, the topical composition of the invention comprises amiloride in a concentration from 0.1 to 30%, preferably in a concentration from 1% to 20%, more preferably in a concentration of 10%, by weight relative to the weight of the composition.

In an embodiment, the topical composition of the invention comprises amiloride in a concentration from 0.1 to 30%, preferably in a concentration of from 1% to 20%, more preferably in a concentration of 10%, by weight relative to the weight of the composition in a base of Excipial Hydrocrème^{®}.

In an embodiment, the topical composition of the invention comprises amiloride in a concentration from 0.1 to 30%, preferably in a concentration of from 1% to 20%, more preferably in a concentration of 10%, by weight relative to the weight of the composition in a base of Codexial Obase^{®}.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### EXAMPLES

### Example 1

A patient aged 76 received Ado-trastuzumab emtansine (T-DM1), the first HER2-targeted antibody-drug conjugate to get the approval of the Food and Drug Administration (FDA), for the treatment of her metastatic HER2-positive breast cancer. This treatment induced peripheral sensory neuropathy of her feet and hands with hypoesthesia and proprioceptive disorders.

For more than 2 years, she had repeated falls due to loss of feeling in her feet, cuts on her hands, unnoticed burns, "splinters in the skin following her gardening activities" with infection in the aftermath because she had not realized it.

She complained of often dropping objects held in her hands and of no longer being able to sew due to the impossibility of holding a fine needle.

She was treated with a cream of amiloride 5% applied locally to the hypoesthetic areas twice a day.

This patient regained the sensation of her hands and feet. She now realizes that she is touching something hot, realizes that she has put a splinter in her hand and can therefore remove it immediately and has not cut herself since switching on masterful preparation.

### Example 2

A patient aged 63 suffered from hypoesthesia and proprioceptive disorders with difficulty gripping the foot on the ground, impression of walking on cotton and complained of the impression of having a permanently rolled sock under the feet in addition to a sensation of having edematous toes since he received a treatment with bortezomib in 2016 for treating his myeloma.

He was treated with a cream of amiloride 10% applied locally to the hypoesthetic areas twice a day.

This patient shown an improvement after 1 month of use, telling that he now feels better hot and cold on the soles of his feet. The impression of walking on cotton disappeared as well as the impression of having a permanently rolled sock under the feet. He can now move his toes better because the sensation of edema has disappeared

### Example 3

A patient aged 35 suffering from colon cancer was treated for 4 months with oxaliplatine in December 2019. He developed hypoesthesia with lost sensations in feet and in the fingertips of both hands and complained of having the impression of a permanently "rolled sock" under the feet.

He was treated with a cream of amiloride 10% applied twice a day during one month and he noticed an improvement in the feeling of the right foot "rolled sock" (4/10 vs 8/10 before this treatment), a little less on the left foot (6/10 vs 8/10). He regained the sensitivity in the fingertips of both hands.

The concentration of amiloride in the cream was increased to 15% and this increase made it possible to further decrease the sensation "rolled sock" even on the left foot.

## Claims

1. A compound selected from amiloride, an amiloride analog and a pharmaceutically acceptable salt thereof, for use in the topical treatment or prevention of a somatosensory impairment.

2. A topical composition comprising a compound selected from amiloride, an amiloride analog and a pharmaceutically acceptable salt thereof, for use in the treatment or prevention of a somatosensory impairment.

3. The topical composition for use according to claim 2, wherein said somatosensory impairment is chosen from the group comprising hypoesthesia and mechanical allodynia.

4. The topical composition for use according to claim 2 or 3, wherein said somatosensory impairment is caused by a neuropathy, traumatic injuries, infections or a needle injection around a nerve.

5. The topical composition for use according to claim 4, wherein said neuropathy is a diabetic neuropathy, a chemotherapy-induced peripheral neuropathy or drug induced peripheral neuropathy,

6. The topical composition for use according to any one of claims 2-5, wherein hypoesthesia affects sensation of pain, temperature, touch, vibration or proprioception

7. The topical composition for use according to any one of claims 2-6, wherein the compound is in a concentration from 0.1 to 30%, in particular from 1% to 20%, more particularly from 10% to 20%, by weight relative to the weight of the composition.

8. The topical composition for use according to any one of claims 2-7, wherein said compound is amiloride or a pharmaceutically salt thereof.

9. The topical composition for use according to any one of claims 2-8, wherein the topical composition is formulated into unit dose form.

10. The topical composition for use according to any one of claims 2-9, wherein the topical composition is applied to the affected area once daily, twice daily, three times daily, once every second day, three times weekly, twice weekly or once weekly.

11. The topical composition for use according to any one of claims 2-10, further comprising a penetration enhancer.
